# EUROPEAN PATENT SPECIFICATION

(11) **EP 2 263 714 B1**
(45) Date of publication and mention of the grant of the patent: **23.04.2014**
(21) Application number: 10011559.1
(22) Date of filing: 13.01.2004
(51) Int. Cl.: A61M 1/16, A61M 1/36, F16L 37/02, A61M 39/10

(54) **Integrated module for blood treatment comprising a support element**
Integriertes Modul zur Blutbehandlung mit einem Stützelement
Module integre en vue d'un traitement sanguin comprenant un element support

(30) Priority: 07.02.2003 IT MI20030214
(43) Date of publication of application: 22.12.2010
(62) Divisional of application: 04701642.3
(73) Proprietor: Gambro Lundia AB, 220 10 Lund (SE)
(72) Inventor: Duchamp, Jacques, 69500 Bron (FR); Aberkane, Aziz, 69150 Decines (FR); Pouchoulin, Dominique, 01390 Tramoyes (FR); Meyssonnier, Gabriel, 38460 Dimizieu (FR)
(74) Representative: Ponzellini, Gianmarco

(56) References cited:
- EP-A- 0 611 227
- EP-A- 0 887 100
- WO-A-01/08722
- DE-A1- 2 907 832
- US-A- 4 436 620

## Description

The invention relates to an integrated module for blood treatment comprising a support element, and to an apparatus for extracorporeal treatment of blood equipped with the integrated module. The invention also relates to a manufacturing process for an integrated module for blood treatment.

As is known, for carrying out extracorporeal blood treatments, such as, for example, hemodialysis, hemofiltration, hemodiafiltration, plasmapheresis, extracorporeal blood oxygenation, extracorporeal blood filtration or other treatments, there must be present at least one extracorporeal circuit through which the blood is made to circulate in order to be transported towards a treatment device. The treated blood is then returned to the patient's cardiovascular system.

With reference, by way of example, to a dialysis treatment, the extracorporeal circuit used comprises: a dialysis filter constituted by a container body including at least a first and a second chamber, separated from each other by a semipermeable nembrane, a blood withdrawal line leading to the first chamber of the dialyzer filter and a blood return line destined to receive blood outletting from the first chamber and to return it to the patient. The second chamber of the dialyzer filter is connected to a dialysis liquid circulation circuit destined to receive the impurities present in the blood, as well as the excess fluid which is to be removed from the patient's blood.

At present, in extracorporeal blood treatment apparatus, the totality of lines destined for dialysis liquid circulation is housed inside the dialysis machine, while the lines forming the extracorporeal blood circuit are changed after each single treatment and are connected to the dialyzer filter, which can be changed either at each treatment or periodically, according to needs.

From the structural point of view, the dialyzer filter, the dialysis liquid circulation lines and the lines forming the patient's blood withdrawal and return branches are constituted by separate parts which are connected up and cooperate operatively following assembly.

The market offers apparatus, in particular destined for intensive kidney failure treatment, which are advantageously provided with integrated modules comprising a support structure, a dialyzer filter constrained to the support structure by means of a support element emerging from the support structure, as well as a hydraulic circuit comprising the tubing necessary for defining the withdrawal branch and the return branch of the blood from and to the patient, the lines (if present) for infusion of anticoagulant, or of substitution liquids, the dialysis liquid supply line, the discharge line for discharge liquid outletting from the dialysis filter second chamber.

A device according to document US 4436620 is an example of the above described prior art; this document discloses all the features according to the preamble of claim 1 and the assembling steps according to the preamble of claim 12.

Another example of the prior art is given by document EP 0611227; this document discloses all the features according to the preamble of claim 1 and the assembling steps according to the preamble of claim 12.

The above-described integrated modules enable an easy and immediate attachment of the lines on the treatment apparatus and do not need any connection between the treatment device, for example a dialyzer filter, and the various tubes or lines destined to carry blood and other fluids. Further, the integrated modules enable removal both of the tubes carrying the blood and those carrying other fluids once the treatment has been concluded. In other words, with a simple loading operation and a connecting-up of the terminals and the fluid transport lines to the relative sources, i.e. bags or other, the user can start up the dialysis apparatus.

Similarly, once the treatment has been concluded, a small number of disconnecting operations and dismounting of the integrated module from the blood treatment machine will enable the operator to completely remove the extracorporeal circuit, the blood treatment device, any tubing for circulation of infusion liquids as well as for the dialysis liquid.

The ease with which the module can be set up guarantees efficiency and speed, much to be appreciated in the case of intensive treatment, where the personnel involved, not necessarily expert in the use of blood treatment machines, can operate quickly and extremely reliably.

Though the above-described integrated modules have had a notable market success, they have shown themselves to be susceptible to improvement in various aspects.

Firstly, in the prior art, the connection between the support body and the blood treatment device includes an additional support interpositioned between the body of the treatment device and the support element, considerably complicating the overall structure of the integrated model.

The presence of an intermediate support structure between the support bcdy and the dialyzer body causes the integrated module to be considerably unwieldy.

Additionally, the need to connect the dialyzer filter or another treatment device used with the extracorporeal blood circuit lines and the treatment fluid lines constitutes a further difficulty, as the connecting-up operations have to be performed in a zone which is difficult to access.

The above has obviously hampered the possibility of automating the assembly stages, considerably increasing production costs of the integrated modules at present on the market.

### SUMMARY OF THE INVENTION

A main aim of the present invention is to make available an integrated module for blood treatment comprising a support element, which overcome all of the above-described limitations and drawbacks.

In particular, an aim of the present invention is to provide a new support element which is easily and automatically assemblable with a blood treatment device, consequently reducing the overall costs for realization of an integrated module for extracorporeal blood treatment.

These aims and more besides will better emerge from the detailed description that follows, of a support element and an integrated module for blood treatment, comprising the support element as characterized in one or more of the appended claims.

According to a first aspect an integrated module for blood treatment according to claim 1 is provided.

In a second aspect in accordance with the first aspect the said base body comprises a frontal wall and a perimeter wall, which perimeter wall is connected by a side thereof to the frontal wall and defines a works area within which at least a portion of a fluid distribution circuitry destined to be associated to the support element can be housed.

In a third aspect in accordance with the second aspect the support element comprises an auxiliary structure extending laterally and externally with respect to the said works housing area from a base zone of the perimeter wall, the said connectors emerging from the said auxiliary structure.

According to a fourth independent aspect, in accordance with claim 1 the integrated module for fluid treatment comprises:
- a support element;
- at least one blood treatment device engaged on the support element and having a ccuple of counter-connectors;
- a fluid distribution circuitry associated to the support element (4) and cooperating with the blood treatment device the support element (4) comprising:
   - a base body (6);
   - at least a first and a second connectors (7, 8) associated to the base body (6) and distanced one from another, receiving and engaging with the corresponding counter-connectors (9, 10) of the blood treatment device (5) mounted on the support element (4), wherein each of the said connectors (7, 8) affords a fluid passage having a first end portion (12), placed in fluid communication with a corresponding channel (13) in the respective counter-connector (9, 10) on the blood treatment device (5), and a second end portion (14), placed in fluid communication with a fluid distribution circuitry (15) associated to the base body (6), each of the said connectors (7, 8) comprising:
   - a tubular channel (16), defining the said first end portion (12),
   - a sealing collar (17), set in a radially external position with respect to the tubular channel(16), and
a connecting wall (18), developing continuously between an external lateral surface (19) of the said tubular channel (16) and an internal lateral surface(20) of the said sealing collar (17) to define an annular seating (21) for engagement of each counter-connector (9, 10).

In a fifth aspect in accordance with the fourth aspect the said blood treatment device is fixed to the base body by at least a pair of the said connectors.

In a sixth aspect in accordance with the fifth aspect the said pair of connectors is interpositioned between the counter-connectors and a portion of the fluid distribution circuitry.

In a seventh aspect in accordance with the fourth aspect the integrated module comprises the support element of the first aspect, wherein the said blood treatment device comprises:
- a containment body;
- at least one semi-permeable membrane operating internally of the containment body and defining a first chamber and a second chamber;
- the first counter-connector and the second counter-connector, associated to the containment body and fixed to respective connectors associated to the base body, at least one of the first counter-connector and the second counter-connector being placed in fluid communication with the second chamber of the blood treatment device and with respective first end portions of the said connectors;
- at least one inlet port to the first chamber; and
- at least one outlet port from the first chamber.

In a eighth aspect in accordance with the seventh aspect the fluid distribution circuitry comprises at least one discharge line of a discharge fluid, placed in communication with the second end portion of one of the said connectors.

In a ninth aspect in accordance with the eighth aspect the fluid distribution circuitry comprises at least one fresh dialysis liquid supply line, placed in communication with the second end portion of another of the connectors.

In a tenth aspect in accordance with the fourth aspect the fluid distribution circuitry comprises at least one blood circuit line having a blood withdrawal branch, placed in communication with the inlet port of the first chamber, and at least one blood return branch, placed in communication with the outlet port of the first chamber.

In a eleventh aspect in accordance with the eighth, ninth or tenth aspect at least one of the said lines is constrained to the support element, defining at least one tract of tubing which is U-shaped in relation to the support element and which is destined during operation to cooperate with a peristaltic pump.

In a twelfth aspect in accordance with the eleventh aspect the at least one U-shaped tract of tubing extends internally or externally with respect to the perimeter wall of the support element.

According to a thirteenth independent aspect an assembly process for an integrated module for fluid treatment according to claim 12 is provided.

In a fourteenth aspect in accordance with the thirteenth aspect during the said insertion stage, at least one portion of the prefixed quantity of glue is arranged in the said second zone of the respective annular seating.

In a fifteenth aspect in accordance with the fourteenth aspect at an end of the said insertion stage, a volume of the said prefixed quantity of glue added to a volume of the portion cf counter-connector housed in the annular seating is less than a total volume of the annular seating.

In a sixteenth aspect in accordance with the thirteenth aspect the stage of associating a fluid distribution circuitry to the support element and to the blood treatment device comprises sub-stages of:
- liquid-proof fixing of an end portion of a discharge line of a discharge fluid with the second end portion of one of the said connectors;
- sealedly fixing an end portion of a fresh dialysis liquid supply line to the second end portion of another of the said connectors;
- sealedly fixing an end portion of a blood withdrawal branch to the inlet port of the first chamber, and an end portion of a blood return branch to the outlet port of the first chamber.

Further characteristics and advantages will better emerge from the detailed description that follows of some preferred embodiments of a support element and an integrated module incorporating the support element of the present invention.

The following detailed description will also illustrate a manufacturing process of an integrated module for blood treatment, according to the invention.

### BRIEF DESCRIPTION OF THE DRAWINGS

The detailed description will now be made with reference to the accompanying figures of the drawings, provided as a non-limiting example, in which:
- Figure 1 is a perspective view of a support element for an integrated module according to a first embodiment of the invention;
- Figure 2 shows the support element of figure 1 in an upturned position relative to the position of figure 1;
- Figure 3 is a perspective view of a cover for closing an open side of the support element of figure 1;
- Figure 4 is a perspective view of a support element for an integrated module in a second embodiment of the invention;
- Figure 5 shows the support element of figure 4 in an upturned position relative to the position of figure 4;
- Figure 6 is a perspective view of an integrated module for extracorporeal treatment of blood with the support element of figure 5;
- Figure 7a shows a detail of the mcdule of figure 6, relating to a coupling between a seating of the support element and a corresponding connector of a blood treatment device;
- Figure 7b shows a detail of figure 1;

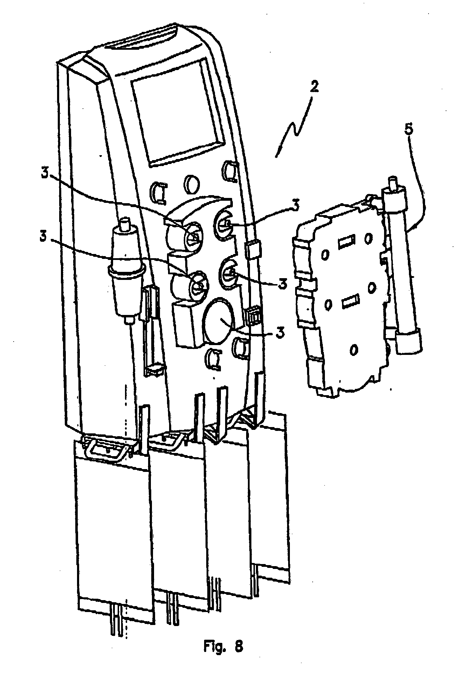

- Figure 8 schematically illustrates the module of figure 6 constrained on a frontal operative wall of a machine for extracorporeal blood treatment;
- Figure 9 is a schematic representation of the module of figure 6 in CRRT (continuous renal replacement therapy) configuration.

### DETAILED DESCRIPTION

With reference to the accompanying figures of the drawings, 1 denotes in its entirety an integrated module for blood treatment in accordance with the present invention. The module 1 can be engaged to a machine for extracorporeal blood treatment 2, provided with one or more pumps 3 destined to cooperate with the module 1. The module 1 comprises a support element 4 to which a blood treatment device 5, for example a plasma-filter, a hemodialysis filter, a hemofiltration filter, a filter for hemodiafiltration or a different unit.

In greater detail, the support element 4 comprises a base body 6 exhibiting at least a first and at least a second connector 7 and 8, distanced one from another, destined to receive and connect with corresponding counter-connectors 9 and 10 of the blood treatment device 5. The first and second connectors 7 and 8 are directly constrained to the base body 6; in the illustrated embodiments the connectors 7 and 8 are made of a rigid plastic material and in a single piece with the base body 6.

The support element 4 exhibits a third connector 11, distanced from the connectors 7, 8 and directly constrained on the base body 6, in the illustrated embodiments the third connector 11 is made of rigid plastic material and in a single piece with the base body 6; the three connectors define pairs of connectors having differentiated interaxes for engaging with corresponding pairs of counter-connectors associated to different blood treatment devices which are mountable on the support element 4. This is so that a single base body 6 can be used to realize integrated modules having different characteristics, thanks to the possibility of engaging treatment devices 5 which are not only different as regards the membrane, but also in terms of overall size and therefore interaxes of the relative counter-connectors.

Each of the connectors 7, 8, 11 constitutes a rigid support and defines a fluid passage having a first end portion 12, destined to be placed in fluid communication with a corresponding channel 13 present in a respective counter-connector 9, 10 exhibited by the blood treatment device 5; each connector 7, 8, 11 also exhibits a second end portion 14, destined to be placed in fluid communication with a fluid distribution circuitry 15 associable to the base body 6. In a further structural detail, each of the connectors 7, 8, 11 comprises a tubular channel 16, defining the first end portion 12, a sealing collar 17, in a position which is radially external to the tubular channel 16, and a connecting wall 18, which develops continuously between an external lateral surface 19 of the tubular chancel 16 and an internal lateral surface 20 of the sealing collar 17.

The external lateral surface 19 of the tubular channel 16, the internal lateral surface 20 of the sealing collar 17 and the connecting wall 18 together define an annular seating 21, a bottom cf which is delimited by the connecting wall 18, shaped in order to receive and engage a corresponding counter-connector of the blood treatment device.

The tubular channel 16 is coaxially arranged with respect to the sealing collar 17, and has geometry of revolution there-with, with a common axis of symmetry. The annular seating 21 exhibits an increasing radial dimension as it progresses from the bottom connecting wall 18, it comprises a first zone 22, adjacent to the bottom and having a constant radial dimension; a second zone 23, distal with respect to the bottom and having a constant radial dimension which is greater than the radial dimension of the first zone 22; and a third zone 24, which is a transit zone between the first and second zones 22 and 23 and which has a progressively growing radial dimension as it progresses away from the bottom connecting wall 18.

The tubular channel 16 and the sealing collar 17 of each connector 7, 8, 11 are parallel to one another in the base body 6, defining a single coupling direction with the corresponding counter-connectors of a treatment device 5.

In the illustrated embodiments, the various counter-connectors and connectors exhibit axes of symmetry which are perpendicular to a frontal wall 25 of the support element 4.

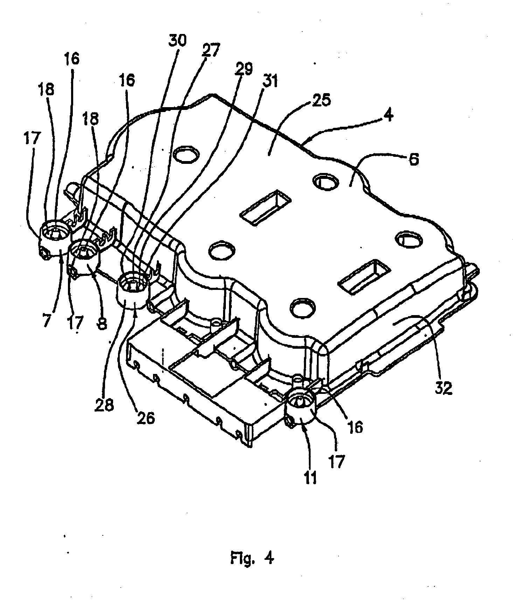

The support element 4 shown in figures 4-7 further comprises a fourth connector 26 which is distanced from the first, second and third connectors 7, 8 and 11. The fourth connector 26 is also directly connected to the support element 4.

In the embodiment of figures 4-7 the fourth connector 26 is made of rigid plastic in a single piece with the base body 6 and defines, with at least one of the other connectors 7, 8 and 11 a further pair of connectors which can be engaged to a corresponding pair of counter-connectors associated to a blood treatment device mountable to the support element 4.

The fourth connector 26 comprises a central cylindrical body 27 for positioning, a sealing collar 28 located in a radially external position with respect to the central cylindrical body 27, and a bottom connecting wall 29 which develops continuously between an external lateral surface 30 of the central cylindrical body 27 and an internal lateral surface 31 of the collar 28. The fourth connector 26 defines a connecting and sealing site for a counter-connector of the blood treatment device 5.

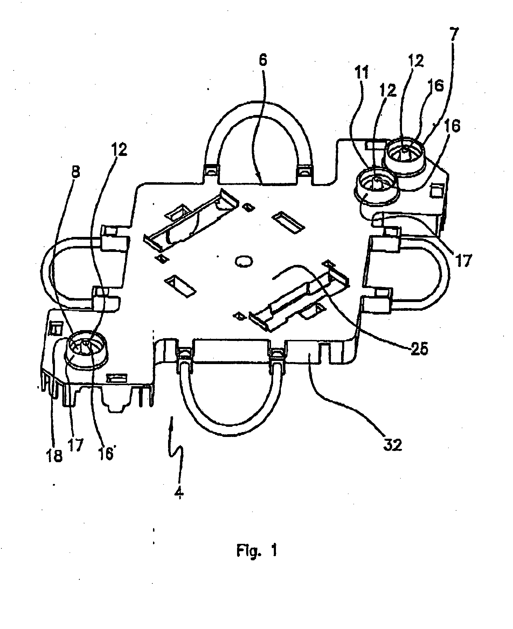

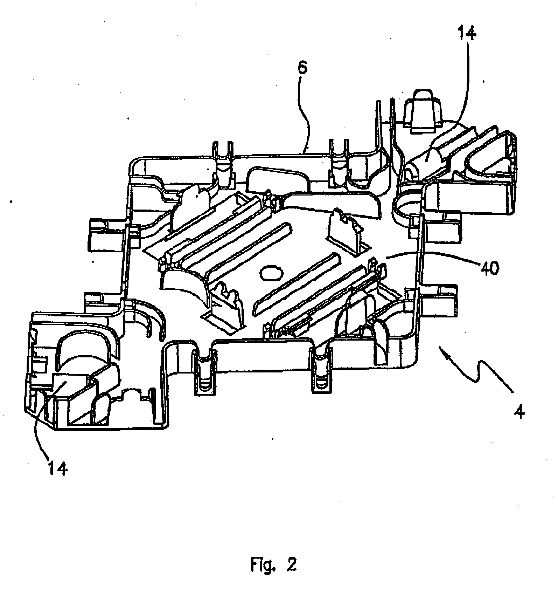

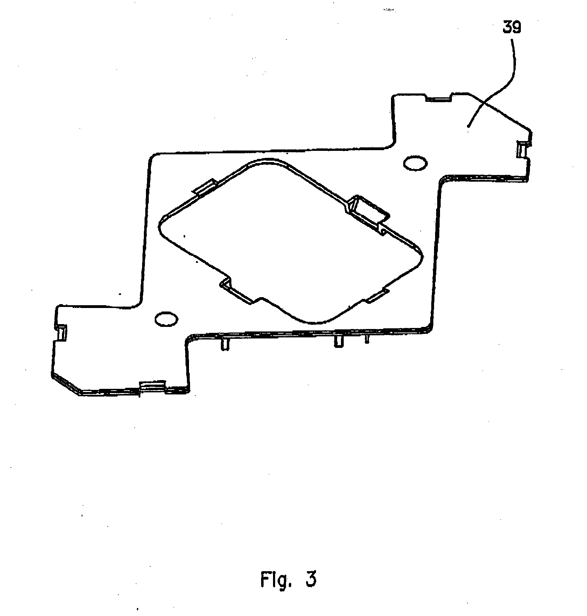

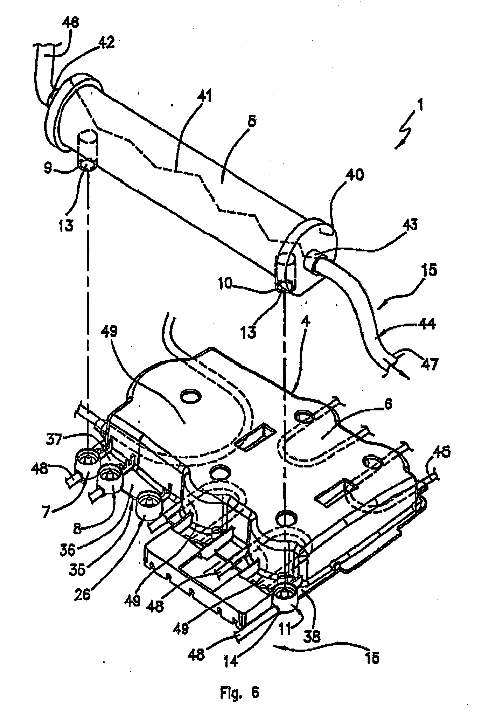

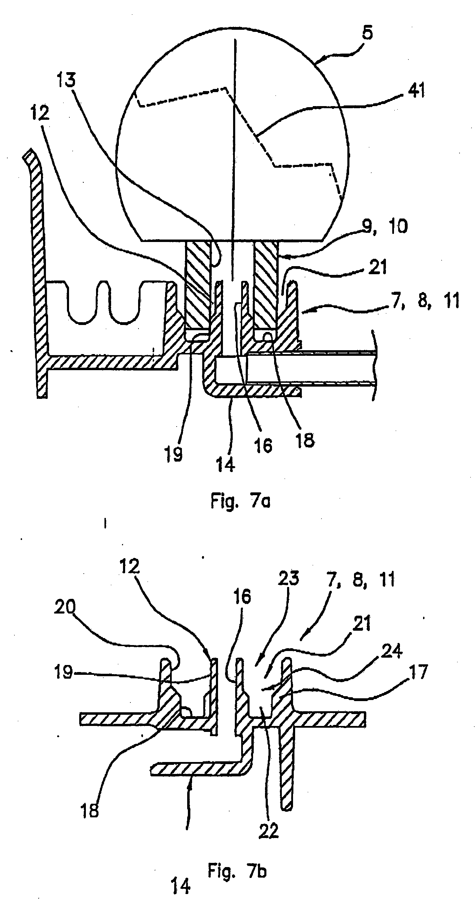

As shown in figures 6, 7a (and the same goes for the support elements of figures 1-3, 7b), the various connectors are made of rigid material in order to offer a mechanical support to the blood treatment device and, according to each individual case, to define a passage or an obstruction for fluid passing through the counter-connectors 9, 10.

In the support element of figures 4-6 the four connectors are aligned and arranged on one side of the base body 6. More precisely, the base body 6 of the element illustrated in figures 4-6 and 7a comprises a frontal wall 25 and a perimeter wall 32 connected around an edge thereof to the frontal wall 25, which together define a works housing area 33 which can house at least a portion of the works of the support element, i.e. a fluid distribution circuitry 15 destined to be associated to the support element 4.

The works housing area 33 exhibits an open side 34 which enables the integrated module 1 to be correctly positioned and adequately locked onto the machine, as will be better described herein below.

The support element 4 exhibits an auxiliary structure 35 which extends laterally and externally with respect to the works housing area 33 from a base zone 36 of the perimeter wall 32. The four connectors emerge from the auxiliary structure 35: the first, second and fourth connector 7, 8, 26 are adjacently situated, and arranged at a first end zone 37 of the auxiliary structure 35, while the third connector 11 is located at a second end zone 38, opposite the first end zone 37.

In the illustrated embodiment of figures 1-3, the base body 6 comprises a frontal wall 25 and a perimeter wall 32 joined at an edge thereof to the frontal wall 25, defining a works housing area 33 which can house at least a portion of the fluid distribution circuitry 15 destined to be associated to the support element 4.

In this embodiment, however, the connectors 7, 8 and 11 are not aligned and emerge directly from the frontal wall 25. Further, a cover 39 is associated to the perimeter wall 32 on an opposite edge thereof with respect to the frontal wall 25.

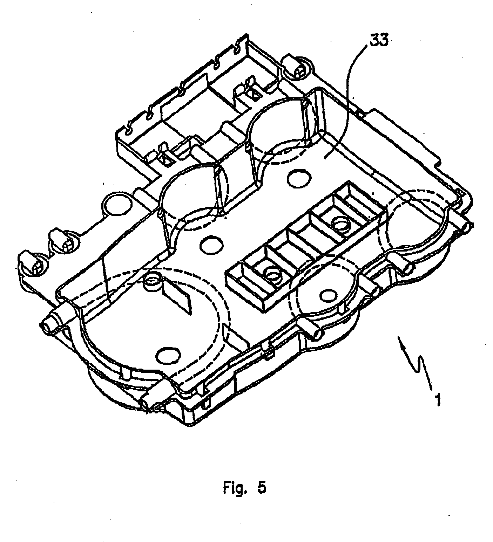

A support element according to the invention can advantageously be used for realizing an integrated module, such as for example the module illustrated in figure 6, where by way of example the support element 4 of figures 4 and 5 is used.

As can be observed, the blood treatment device 5 is fixed to the support element 4 by at least one pair of connectors; the blood treatment device comprises a body 40, at least one semipermeable membrane 41 (for example a parallel hollow fiber membrane or a plate membrane) operating internally of the bcdy 40 and defining a first chamber and a second chamber; a first and a second connector are associated to the body 40 and fixed to the respective connectors on the base body 6.

The first and second counter-connectors 9, 10, are tubular and are in fluid communication with the second chamber of the treatment device and with respective first end portions 12 of the connectors.

The treatment device exhibits an inlet port 42 to the first chamber, and at least one outlet port 43 from the first chamber, for connection of an extracorporeal blood circuit line 44 or another physiological fluid.

A fluid distribution circuitry 15 is attached to the support element 4 and cooperates with the treatment device 5. In more detail, the circuitry comprises:
- at least one discharge line 45 of discharge fluid, in communication with the second terminal portion 14 of one of the connectors;
- at least one blood line 44 having a blood withdrawal branch 46, placed in communication with the inlet 42 of the first chamber, and at least one branch 47 of a blood return line, placed in communication with the outlet 43 of the first chamber;
- at least one supply line 48 of fresh dialysis liquid, placed in communication with the second end portion 14 of another of the connectors.

Each of the lines is constrained to the support element 4, defining at least one tract of tubing 49 which is arranged in a U-shape, in relation to the support element 4.

During operation the U-shaped tracts are destined to cooperate with the respective peristaltic pumps 3 located on a panel of a machine for extracorporeal blood treatment. Each tract of U-shaped tubing extends internally or externally (figure 7) with respect to the perimeter wall 32 of the support element 4.

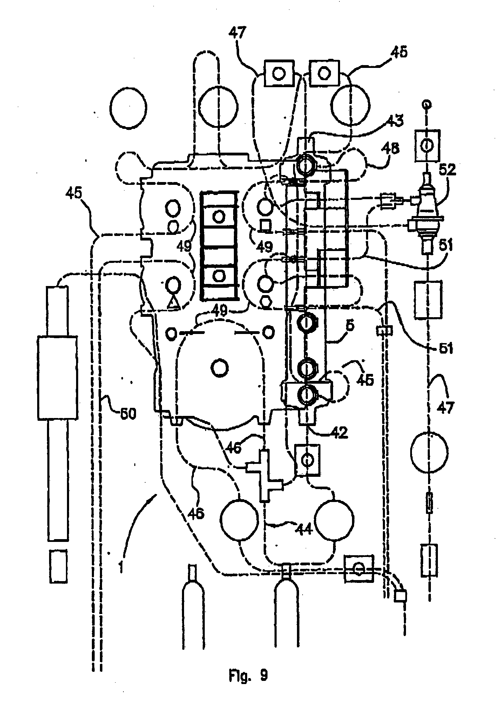

Figure 9 is a diagram of the integrated module 1 in the CRRT configuration. As can be observed, the module 1 also has lines 50 and 51 for respective pre and post blood pump infusions (pre-dilution and/or post-dilution).

An air separator device 52 operates on the branch 47 of the blood line 44 and receives an infusion line 51.

The invention also relates to an assembly procedure for an integrated module for fluid treatment which comprises the stages of predisposing a support element 4, for example such as in figures 1-3 or in figures 4-6, as well as a treatment device 5 which is intended for coupling to the support element 4. The blood treatment device 5 is then fixed to the support element 4. Finally a fluid distribution circuitry 15 is associated to the support element 4 and to the blood treatment device 5 so as to create the necessary blood circulation lines, the discharge lines, the infusion lines for any liquid substitution lines, and dialysis lines.

The connection of the distribution circuitry to the blood treatment device can be done before, at the same time as or after the circuitry fixing stage to the support element 4.

The fixing stage of the blood treatment device to the support element 4 comprises the sub-stages of selecting a pair of connectors to which the counter-connectors 9, 10 on the blood treatment device are to be connected, applying a predetermined quantity of glue, normally polymer-resin based, in the annular seatings 21 of each chosen connector, at least partially inserting each counter-connector in the respective annular seating in order to obtain a mechanical bond and a liquid-proof seal coupling.

During the insertion stage, at least a portion of the glue applied in the annular seating actually settles in the second zone 23 of the annular seating. On completion of the counter-connector insertion stage in the annular seating, the volume of the quantity of glue previously applied added to the volume of the portion of counter-connector housed in the annular seating is less than the overall volume of the annular seating. This prevents any glue material from migrating towards the tubular channel 16 and causing a partial or total occlusion.

The stage of associating a fluid distribution circuitry 15 to the support element 4 and the blood treatment device 5 comprises the sub-stages of liquid-proof sealing of an end portion of a discharge fluid discharge line 45 with the second end portion 14 of one of the connectors, and of sealedly fixing an end portion of a fresh dialysis liquid supply line 48 to the second end portion of a further of the connectors.

The stage of associating the blood distribution circuitry also includes sealedly fixing an end portion of a blood withdrawal branch 46 to an inlet port of the first chamber, and an end portion of a blood return branch 47 to an outlet port of the first chamber.

The fixing of the various above-mentioned end portions can be achieved by gluing, friction fitting or hot-coupling.

The invention provides important advantages.

Firstly, the direct fixing of the blood treatment device to the selected connectors of the support element does not require the use of other support elements of the same device.

Further, the connectors receive on one side the counter-connectors of the blood treatment device and on the other side the end portions of distribution circuitry lines, realizing a contemporaneous mechanical and hydraulic connection between the fluid distribution circuitry and the blood treatment device.

The presence of various connectors means the treatment device can be used with connectors having different interaxes.

The special fixing modality of the blood treatment device and the various fluid lines to the support element considerably facilitates the assembly process of an integrated module according to the invention.

The specific structure of the integrated module and the support element minimises the length of fluid line needed to realize the connections with the blood treatment device.

## Claims

1. An integrated module (1) for fluid treatment, comprising:
- a support element (4);
- at least one blood treatment device (5) engaged on the support element (4) and having a couple of counter-connectors (9, 10);
- a fluid distribution circuitry (15) associated to the support element (4) and cooperating with the blood treatment device (5), the support element (4) comprising:
- a base body (6);
**characterized in that** the support element (4) further comprises:
- at least a first and a second connectors (7, 8) associated to the base body (6) and distanced one from another, receiving and engaging with the corresponding counter-connectors (9, 10) of the blood treatment device (5) mounted on the support element (4), wherein each of the said connectors (7, 8) affords a fluid passage having a first end portion (12), placed in fluid communication with a corresponding channel (13) in the respective counter-connector (9, 10) on the blood treatment device (5), and a second end portion (14), placed in fluid communication with a fluid distribution circuitry (15) associated to the base body (6), each of the said connectors (7, 8) comprising:
- a tubular channel (16), defining the said first end portion (12),
- a sealing collar (17), set in a radially external position with respect to the tubular channel (16), and
- a connecting wall (18), developing continuously between an external lateral surface (19) of the said tubular channel (16) and an internal lateral surface (20) of the said sealing collar (17) to define an annular seating (21) for engagement of each counter-connector (9, 10).

2. The integrated module of claim 1, wherein the tubular channel (16) defining the said first end portion (12) is coaxially arranged with respect to the sealing collar (17), the said annular seating (21) exhibiting a bottom which is delimited by the said connecting wall (18).

3. The integrated module of claim 2, wherein the said annular seating (21) exhibits a radial dimension which increases progressively in a direction moving away from the said bottom wall (18).

4. The integrated module of claim 3, wherein the said annular seating (21) exhibits: a first zone (22), adjacent to the said bottom wall (18) and having a constant radial dimension; a second zone (23), distal of the said bottom wall (18) and having a constant radial dimension which is greater than the radial dimension of the first zone (22); and a third zone (24), which is a transition zone between the first zone (22) and the second zone (23) and has a progressively increasing dimension in a distancing direction from the said bottom wall (18).

5. The integrated module of claim 1, wherein the tubular channel (16) and the sealing collar (17) of each connector (7, 8) are parallel to one another as they emerge from the base body (6), defining a single coupling direction for coupling with the corresponding counter-connectors (9, 10) of the blood treatment device (5).

6. The integrated module of claim 1, wherein the said base body (6) comprises a frontal wall (25) and a perimeter wall (32), which perimeter wall (32) is connected by a side thereof to the frontal wall (25) and defines a works area (33) within which at least a portion of a fluid distribution circuitry (15) associated to the support element (4) is housed.

7. The integrated module of claim 6, comprising an auxiliary structure (35) extending laterally and externally with respect to the said works housing area (33) from a base zone (36) of the perimeter wall (32), the said connectors (7, 8) emerging from the said auxiliary structure (35).

8. The integrated module of claim 1, wherein the said pair of connectors is interpositioned between the counter-connectors (9) and (10) and a portion of the fluid distribution circuitry (15).

9. The integrated module of claim 1, wherein the said blood treatment device (5) comprises:
- a containment body (40);
- at least one semi-permeable membrane (41) operating internally of the containment body (40) and defining a first chamber and a second chamber;
- the first counter-connector (9) and the second counter-connector (10), associated to the containment body (40) and fixed to respective connectors (7), (8) and (11) associated to the base body (6), at least one of the first counter-connector and the second counter-connector being placed in fluid communication with the second chamber of the blood treatment device (5) and with respective first end portions (12) of the said connectors;
- at least one inlet port (42) to the first chamber; and
- at least one outlet port (43) from the first chamber.

10. The integrated module of claim 1, wherein the fluid distribution circuitry (15) comprises at least one discharge line (45) of a discharge fluid, placed in communication with the second end portion (14) of one of the said connectors (7, 8), in particular
the fluid distribution circuitry (15) comprising at least one fresh dialysis liquid supply line (48), placed in communication with the second end portion (14) of another of the connectors (7, 8) and wherein the fluid distribution circuitry (15) comprises at least one blood circuit line (44) having a blood withdrawal branch (46), placed in communication with the inlet port (42) of the first chamber, and at least one blood return branch (47), placed in communication with the outlet port (43) of the first chamber,

11. The integrated module of claim 10, wherein at least one of the said lines (44, 45, 48) is constrained to the support element (4), defining at least one tract of tubing (49) which is U-shaped in relation to the support element (4) and which is destined during operation to cooperate with a peristaltic pump (3), the U-shaped tract of tubing (49) extending internally or externally with respect to the perimeter wall (32) of the support element (4).

12. An assembly process for an integrated module (1) for fluid treatment comprising stages of:
- predisposing a support element (4);
- fixing a blood treatment device (5) to the support element (4);
**characterized in that** the support element is according to any one of the preceding claims and **in that** the fixing stage comprises sub-stages as follows:
- selecting of a pair of connectors (7, 8) to which the counter-connectors (9, 10) of the blood treatment device (5) are to be fixed;
- depositing a prefixed quantity of glue in the annular seatings (21) of each connectors which has been selected;
- at least partially inserting each counter-connector (9, 10) into a respective annular seating (21) in order to obtain a mechanical lock and a liquid-proof seal;
- associating a fluid distribution circuitry (15) to the support element (4) and to the blood treatment device (5).

13. The process of claim 12, wherein during the said insertion stage, at least one portion of the prefixed quantity of glue is arranged in the said second zone (23) of the respective annular seating (21).

14. The process of claim 13, wherein at an end of the said insertion
stage, a volume of the said prefixed quantity of glue added to a volume of the portion of counter-connector (9, 10) housed in the annular seating (21) is less than a total volume of the annular seating.

15. The process of claim 12, wherein the stage of associating a fluid distribution circuitry (15) to the support element (4) and to the blood treatment device (5) comprises sub-stages of:
- liquid-proof fixing of an end portion of a discharge line (45) of a discharge fluid with the second end portion (14) of one of the said connectors (7, 8);
- sealedly fixing an end portion of a fresh dialysis liquid supply line (48) to the second end portion (14) of another of the said connectors (7, 8);
- sealedly fixing an end portion of a blood withdrawal branch (46) to the inlet port (42) of the first chamber, and an end portion of a blood return branch (47) to the outlet port (43) of the first chamber.

## Patentansprüche

1. Integriertes Modul (1) zur Fluidbehandlung umfassend:
- ein Tragelement (4);
- mindestens eine Blutbehandlungsvorrichtung (5), die am Tragelement (4) eingreift und ein Paar von Gegenverbindern (9, 10) besitzt,
- einen Fluidverteilungskreislauf (15), der mit dem Tragelement (4) verbunden ist und mit der Blutbehandlungsvorrichtung (5) zusammenwirkt, das Tragelement (4)umfassend:
- einen Basiskörper (6);
**dadurch gekennzeichnet, dass** das Tragelement (4) weiter umfasst:
- mindestens einen ersten und einen zweiten Verbinder (7, 8), die mit dem Basiskörper (6) verbunden und voneinander beabstandet sind und die die entsprechenden Gegenverbinder (9, 10) der Blutbehandlungsvorrichtung (5), die am Tragelement (4) montiert ist, empfangen und mit diesen eingreifen, wobei jeder der Verbinder (7, 8) einen Fluiddurchgang mit einem ersten Endabschnitt (12), der mit einem entsprechenden Kanal (13) im entsprechenden Gegenverbinder (9, 10) an der Blutbehandlungsvorrichtung (5) in Fluidverbindung gesetzt ist, und einem zweiten Endabschnitt (14), der mit einem mit dem Basiskörper (6) verbundenen Fluidverteilungskreislauf (15) in Fluidverbindung gesetzt ist, bereitstellt, jeder der Verbinder (7, 8) umfassend:
- einen Rohrkanal (16), der den ersten Endabschnitt (12) definiert,
- eine Dichtmanschette (17), der in einer radial äußeren Stellung zum Rohrkanal (16) angeordnet ist, und
- eine Verbindungswand (18), die sich kontinuierlich zwischen einer äußeren Seitenfläche (19) des Rohrkanals (16) und einer inneren Seitenfläche (20) der Dichtmanschette (17) entwickelt, um eine Ringaufnahme (21) zum Eingreifen jedes Gegenverbinders (9, 10) zu definieren.

2. Integriertes Modul nach Anspruch 1, wobei der Rohrkanal (16), der den ersten Endabschnitt (12) definiert, koaxial gegenüber der Dichtmanschette (17) angeordnet ist, wobei die Ringaufnahme (21) einen Boden besitzt, der von der Verbindungswand (18) begrenzt ist.

3. Integriertes Modul nach Anspruch 2, wobei die Ringaufnahme (21) eine radiale Größe aufweist, die sich in einer Richtung weg von der Bodenwand (18) zunehmend vergrößert.

4. Integriertes Modul nach Anspruch 3, wobei die Ringaufnahme (21) aufweist: einen ersten Bereich (22), der an der Bodenwand (18) anliegt und eine konstante radiale Größe besitzt; einen zweiten Bereich (23), distal von der Bodenwand (18) angeordnet und eine konstante radiale Größe besitzend, die größer ist als die radiale Größe des ersten Bereichs (22); und einen dritten Bereich (24), der ein Übergangsbereich zwischen dem ersten Bereich (22) und dem zweiten Bereich (23) ist und eine zunehmend größer werdende Größe in einer Richtung weg von der Bodenwand (18) besitzt.

5. Integriertes Modul nach Anspruch 1, wobei der Rohrkanal (16) und die Dichtmanschette (17) jedes Verbinders (7, 8) zueinander parallel sind, wo sie aus dem Basiskörper (6) herausragen, eine einzige Kopplungsrichtung zum Koppeln mit entsprechenden Gegenverbindern (9, 10) der Blutbehandlungsvorrichtung (5) definierend.

6. Integriertes Modul nach Anspruch 1, wobei der Basiskörper (6) eine Vorderwand (25) und eine Umfangswand (32) umfasst, wobei die Umfangswand (32) durch eine ihrer Seiten mit der Vorderwand (25) verbunden ist und eine Arbeitsfläche (33) definiert, in dem mindestens ein Abschnitt eines Fluidverteilungskreislaufs (15), der mit dem Tragelement (4) zu verbinden ist, untergebracht ist.

7. Integriertes Modul nach Anspruch 6, umfassend eine Nebenstruktur (35), die sich seitlich und außen in Bezug auf die Arbeitsaufnahmefläche (33) von einem Basisbereich (36) der Umfangswand (32) erstreckt, wobei die Verbinder (7, 8) von der Nebenstruktur (35) herausragen.

8. Integriertes Modul nach Anspruch 1, wobei das Verbinderpaar zwischen den Gegenverbindern (9) und (10) und einem Abschnitt des Fluidverteilungskreislaufs (15) zwischengeordnet ist.

9. Integriertes Modul nach Anspruch 1, wobei die Blutbehandlungsvorrichtung (5) umfasst:
- einen Kapselungskörper (40);
- mindestens eine semipermeable Membran (41), die innen im Kapselungskörper (40) wirkt und eine erste Kammer und eine zweite Kammer definiert;
- den ersten Gegenverbinder (9) und den zweiten Gegenverbinder (10), die mit dem Kapselungskörper (40) verbunden und an entsprechenden, mit dem Basiskörper (6) verbundenen Verbindern (7), (8) und (11) befestigt sind, wobei mindestens ein zwischen dem ersten Gegenverbinder und dem zweiten Gegenverbinder mit der zweiten Kammer der Blutbehandlungsvorrichtung (5) und mit entsprechenden ersten Endabschnitten (12) der Verbinder in Fluidverbindung gesetzt ist;
- mindestens eine Eingangsöffnung (42) zur ersten Kammer; und
- mindestens einen Ausgangsöffnung (43) von der ersten Kammer.

10. Integriertes Modul nach Anspruch 1, wobei der Fluidverteilungskreislauf (15) mindestens eine Abflussleitung (45) für ein Abflussfluid umfasst, die mit dem zweiten Endabschnitt (14) eines der Verbinder (7, 8) in Verbindung gesetzt ist, insbesondere wobei der Fluidverteilungskreislauf (15) mindestens eine Zuführungsleitung für frische Dialyseflüssigkeit (48) umfasst, die mit dem zweiten Endabschnitt (14) eines anderen der Verbinder (7, 8) in Verbindung gesetzt ist, und wobei der Fluidverteilungskreislauf (15) mindestens eine Blutkreislaufleitung (44) mit einer Blutentnahmeabzweigung (46), die mit der Eingangsöffnung (42) der ersten Kammer in Verbindung gesetzt ist, und mindestens einer Blutrückführungsabzweigung (47), die mit der Ausgangsöffnung (43) der ersten Kammer in Verbindung gesetzt ist, umfasst.

11. Integriertes Modul nach Anspruch 10, wobei mindestens eine der Leitungen (44, 45, 48) mit dem Tragelement (4) fest verbunden ist, wodurch sie mindestens einen Rohrabschnitt (49) definiert, der U-förmig gegenüber dem Tragelement (4) ist und während des Betriebs mit einer peristaltischen Pumpe (3) zusammenwirken soll, wobei der U-förmige Rohrabschnitt (49) sich innen oder außen in Bezug auf die Umfangswand (32) des Tragelements (4) erstreckt.

12. Montageverfahren für ein integriertes Modul (1) zur Fluidbehandlung umfassend die Schritte:
- Vorbereiten eines Tragelementes (4);
- Befestigen einer Blutbehandlungsvorrichtung (5) am Tragelement (4), **dadurch gekennzeichnet, dass** das Tragelement nach einem der vorherigen Ansprüche ist und dass der Befestigungsschritt die folgenden Unterschritte umfasst:
- Auswählen eines Paars von Verbindern (7, 8), an den die Gegenverbinder (9, 10) der Blutbehandlungsvorrichtung (5) zu befestigen sind;
- Abgeben einer vorbestimmten Menge von Klebemittel in den Ringaufnahmen (21) jedes ausgewählten Verbinders;
- mindestens teilweises Einführen jedes Gegenverbinders (9, 10) in eine entsprechende Ringaufnahme (21), um eine mechanische Verriegelung und eine Flüssigkeitsabdichtung zu erzielen;
- Verbinden eines Fluidverteilungskreislaufs (15) mit dem Tragelement (4) und mit der Blutbehandlungsvorrichtung (5).

13. Verfahren nach Anspruch 12, wobei während des Einführungsschrittes mindestens ein Teil der vorbestimmten Menge von Klebemittel im zweiten Bereich (23) der entsprechenden Ringaufnahme (21) angeordnet wird.

14. Verfahren nach Anspruch 13, wobei am Ende des Einführungsschrittes ein Volumen der vorbestimmten Menge von Klebemittel zuzüglich eines Volumens des Abschnitts des Gegenverbinders (9, 10), der in der Ringaufnahme (21) aufgenommen ist, weniger als ein Gesamtvolumen der Ringaufnahme beträgt.

15. Verfahren nach Anspruch 12, wobei der Schritt umfassend das Verbinden eines Fluidverteilungskreislaufs (15) mit dem Tragelement (4) und mit der Blutbehandlungsvorrichtung (5) die Unterschritte umfasst:
- flüssigkeitsdichtes Befestigen eines Endabschnitts einer Abflussleitung (45) für ein Abflussfluid am zweiten Endabschnitt (14) eines der Verbinder (7, 8);
- abgedichtetes Befestigen eines Endabschnitts einer Zuführungsleitung für frische Dialyseflüssigkeit (48) am zweiten Endabschnitt (14) eines anderen der Verbinder (7, 8);
- abgedichtetes Befestigen eines Endabschnitts einer Blutentnahmeabzweigung (46) an der Eingangsöffnung (42) der ersten Kammer, und eines Endabschnitts einer Blutrückführungsabzweigung (47) an der Ausgangsöffnung (43) der ersten Kammer.

## Revendications

1. Module intégré (1) pour le traitement de fluide, comprenant:
- un élément de support (4);
- au moins un dispositif de traitement du sang (5) engagé sur l'élément de support (4) et ayant une paire de contre-connecteurs (9, 10);
- un circuit de distribution de fluide (15) associé à l'élément de support (4) et coopérant avec le dispositif de traitement du sang (5), l'élément de support (4) comprenant:
- un corps de base (6);
**caractérisé en ce que** l'élément de support (4) comprend en outre:
- au moins un premier et un deuxième connecteur (7, 8) associés au corps de base (6) et placés à une certaine distance l'un de l'autre, aptes à recevoir et s'engager avec des contre-connecteurs correspondants (9, 10) du dispositif de traitement du sang (5) monté sur l'élément de support (4), où chacun desdits connecteurs (7, 8) crée un passage de fluide ayant une première portion d'extrémité (12) mise en communication de fluide avec un canal correspondant (13) dans le contre-connecteur respectif (9, 10) dans le dispositif de traitement du sang (5), et une deuxième portion d'extrémité (14) mise en communication de fluide avec un circuit de distribution de fluide (15) associé au corps de base (6), chacun desdits connecteurs (7, 8) comprenant:
- un canal tubulaire (16) définissant ladite première portion d'extrémité (12),
- un collier d'étanchéité (17) placé dans une position radialement extérieure par rapport au canal tubulaire (16), et
- une paroi de liaison (18) se développant de façon continue entre une surface latérale extérieure (19) dudit canal tubulaire (16) et une surface latérale intérieure (20) dudit collier d'étanchéité (17) pour définir un siège annulaire (21) pour l'engagement de chaque contre-connecteur (9, 10).

2. Module intégré selon la revendication 1, où le canal tubulaire (16) définissant ladite première portion d'extrémité (12) est rangé de façon coaxiale par rapport au collier d'étanchéité (17), ledit siège annulaire (21) ayant un fond délimité par ladite paroi de liaison (18).

3. Module intégré selon la revendication 2, où ledit siège annulaire (21) présente une dimension radiale augmentant progressivement dans une direction s'éloignant de ladite paroi de fond (18).

4. Module intégré selon la revendication 3, où ledit siège annulaire (21) présente:
une première zone (22) adjacente à ladite paroi de fond (18) et ayant une dimension radiale constante; une deuxième zone (23) distale par rapport à ladite paroi de fond (18) et ayant une dimension radiale constante supérieure à la dimension radiale de la première zone (22);
et une troisième zone (24) qui est une zone de transition entre la première zone (22) et la deuxième zone (23) et présente une dimension qui augmente progressivement dans une direction s'éloignant de ladite paroi de fond (18).

5. Module intégré selon la revendication 1, où le canal tubulaire (16) et le collier d'étanchéité (17) de chaque connecteur (7, 8) sont parallèles l'un à l'autre lorsqu'ils sortent du corps de base (6), définissant une seule direction pour l'accouplement avec les contre-connecteurs correspondants (9, 10) du dispositif de traitement du sang (5).

6. Module intégré selon la revendication 1, où ledit corps de base (6) comprend une paroi antérieure (25) et une paroi périmétrale (32), laquelle paroi périmétrale (32) est reliée à travers l'un de ses côtés à la paroi antérieure (25) et définit une zone de travail (33) dans laquelle au moins une portion d'un circuit de distribution de fluide (15) associée à l'élément de support (4) est reçue.

7. Module intégré selon la revendication 6, comprenant une structure auxiliaire (35) s'étendant latéralement et à l'extérieur par rapport à ladite zone de travail de logement (33) d'une zone de base (36) de la paroi périmétrale (32), lesdits connecteurs (7, 8) saillant de ladite structure auxiliaire (35).

8. Module intégré selon la revendication 1, où ladite paire de connecteurs est placée entre les contre-connecteurs (9) et (10) et une portion du circuit de distribution de fluide (15).

9. Module intégré selon la revendication 1, où ledit dispositif de traitement du sang (5) comprend:
- un corps contenant (40);
- au moins une membrane semi-perméable (41) opérant à l'intérieur du corps contenant (40) et définissant un premier compartiment et un deuxième compartiment;
- le premier contre-connecteur (9) et le deuxième contre-connecteur (10) associés au corps contenant (40) et fixés aux connecteurs respectifs (7), (8) et (11) associés au corps de base (6), au moins un entre le premier contre-connecteur et le deuxième contre-connecteur étant mis en communication de fluide avec le deuxième compartiment du dispositif de traitement du sang (5) et avec des premières portions d'extrémité respectives (12) desdits connecteurs;
- au moins un orifice d'entrée (42) au premier compartiment; et
- au moins un orifice de sortie (43) du premier compartiment.

10. Module intégré selon la revendication 1, où le circuit de distribution de fluide (15) comprend au moins un conduit de décharge (45) d'un fluide de décharge, mis en communication avec la deuxième portion d'extrémité (14) de l'un desdits connecteurs (7, 11 ), en particulier le circuit de distribution de fluide (15) comprenant au moins un conduit d'alimentation de liquide de dialyse frais (48), mis en communication avec la deuxième portion d'extrémité (14) de l'autre desdits connecteurs (7, 8), et où le circuit de distribution de fluide (15) comprend au moins un conduit de circuit du sang (44) ayant une ramification de prélèvement du sang (46), mise en communication avec l'orifice d'entrée (42) du premier compartiment, et au moins une ramification de retour du sang (47), mise en communication avec l'orifice de sortie (43) du premier compartiment.

11. Module intégré selon la revendication 10, où au moins un desdits conduits (44, 45, 48) est lié à l'élément de support (4), définissant au moins une portion de conduit (49) en forme de U par rapport à l'élément de support (4) et apte pendant l'usage à coopérer avec une pompe péristaltique (3), la portion de conduit en forme de U (49) s'étend à l'intérieur ou à l'extérieur par rapport à la paroi périmétrale (32) de l'élément de support (4).

12. Procédé de montage pour un module intégré (1) pour le traitement de fluide, comprenant les étapes suivantes:
- préparer un élément de support (4);
- fixer un dispositif de traitement du sang (5) à l'élément de support (4); **caractérisé en ce que** l'élément de support est selon une quelconque des revendications précédentes et que l'étape de fixation comprend les sous-étapes suivantes:
- sélectionner une paire de connecteurs (7, 8) auxquels les contre-connecteurs (9, 10) du dispositif de traitement du sang (5) sont à fixer;
- appliquer une quantité préétablie de colle dans les sièges annulaires (21) de chaque connecteur ayant été sélectionné;
- introduire au moins partiellement chaque contre-connecteur (9, 10) dans un siège annulaire respectif (21) afin d'obtenir un blocage mécanique et une étanchéité au liquide;
- associer un circuit de distribution de fluide (15) à l'élément de support (4) et au dispositif de traitement du sang (5).

13. Procédé selon la revendication 12, où pendant ladite étape d'introduction au moins une partie de la quantité préétablie de colle est placée dans ladite deuxième zone (23) du siège annulaire respectif (21).

14. Procédé selon la revendication 13, où à la fin de ladite étape d'introduction un volume de ladite quantité préétablie de colle plus un volume de la portion de contre-connecteur (9, 10) reçue dans le siège annulaire (21) est inférieur à un volume total du siège annulaire.

15. Procédé selon la revendication 12, où l'étape d'associer un circuit de distribution de fluide (15) à l'élément de support (4) et au dispositif de traitement du sang (5) comprend les sous-étapes suivantes:
- fixer de façon étanche au liquide une portion d'extrémité d'un conduit de décharge (45) d'un fluide de décharge à la deuxième portion d'extrémité (14) de l'un desdits connecteurs (7, 8);
- fixer de façon étanche une portion d'extrémité d'un conduit d'alimentation de liquide de dialyse frais (48) à la deuxième portion d'extrémité (14) de l'autre desdits connecteurs (7, 8);
- fixer de façon étanche une portion d'extrémité d'une ramification de prélèvement du sang (46) à l'orifice d'entrée (42) du premier compartiment, et une portion d'extrémité d'une ramification de retour du sang (47) à l'orifice de sortie (43) du premier compartiment.
